# EUROPÄISCHE PATENTSCHRIFT

(11) **EP 2 103 282 B1**
(45) Veröffentlichungstag und Bekanntmachung des Hinweises auf die Patenterteilung: **02.06.2010**
(21) Anmeldenummer: 09003943.9
(22) Anmeldetag: 19.03.2009
(51) Int. Cl.: A61F 2/18

(54) **Gehörknöchelchenprothese mit variablen Ankopplungsflächen**
Ossicular prosthetic with variable coupling surfaces
Prothèse de l'osselet de l'oreille dotée de surfaces d'accouplement variables

(30) Priorität: 20.03.2008 DE 102008015115
(43) Veröffentlichungstag der Anmeldung: 23.09.2009
(73) Patentinhaber: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE)
(74) Vertreter: Kohler Schmid Möbus

(56) Entgegenhaltungen:
- DE-A1- 19 744 789
- DE-B3-102007 013 708

## Beschreibung

Die Erfindung betrifft eine Gehörknöchelchenprothese, die mindestens ein Glied oder Teile eines Gliedes der Gehörknöchelchenkette ersetzt oder überbrückt, wobei die Gehörknöchelchenprothese an ihrem einen Ende ein im Wesentlichen plattenförmiges erstes Befestigungselement zur Anlage am Trommelfell und an ihrem anderen Ende ein zweites Befestigungselement zur mechanischen Verbindung mit einem Glied oder Teilen eines Gliedes der Gehörknöchelchenkette oder mit dem Innenohr sowie ein die beiden Befestigungselemente Schall leitend miteinander verbindendes Verbindungselement umfasst, und wobei das plattenförmige erste Befestigungselement einen radial inneren, insbesondere um den Flächenschwerpunkt des plattenförmigen ersten Befestigungselements zentral angeordneten Ankoppelbereich zum mechanischen Ankoppeln des ersten Befestigungselements an das Verbindungselement sowie mehrere Stegelemente zur radialen Verbindung des radial inneren Ankoppelbereichs mit radial äußeren Abschnitten des ersten Befestigungselements aufweist, wobei die radial äußeren Abschnitte des plattenförmigen ersten Befestigungselements einen äußeren Ringbereich bilden, der radial äußere Ringbereich mindestens eine, vorzugsweise mehrere Unterbrechungen aufweist.

Eine derartige Vorrichtung ist bekannt aus der DE 10 2007 013 708 B3, die die Merkmale des Oberbegriffs des Anspruchs 1 zeigt.

Gehörknöchelchenprothesen werden verwendet, um bei ganz oder teilweise fehlenden oder geschädigten Gehörknöchelchen des menschlichen Mittelohrs den Schall bzw. das Schallsignal vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung bzw. Signalübertragung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Nachdem die Prothese operativ im Mittelohr platziert wurde und das Trommelfell wieder verschlossen ist, beginnt die so genannte Einheilungsphase. In dieser Zeit bilden sich Narben und Gewebestränge und diese verursachen unvorhersehbare Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse postoperative Mobilität und Flexibilität aufweist, so dass sich die Kopfplatte nach der Operation selbstständig der Position des Trommelfells angleichen kann.

Da zudem die anatomischen Gegebenheiten des Ohrs, wie beispielsweise die Lage, die Form und die Größe des Steigbügels, des Ambosses, des Hammers und des Trommelfells von Mensch zu Mensch variieren, ist es sehr vorteilhaft, wenn Gehörknöchelchenprothesen nicht starr ausgebildet sind, sondern eine gewisse Flexibilität oder Variabilität aufweisen.

Um eine solche Flexibilität/Variabilität zu erreichen sind verschiedene Befestigungs- und Ankopplungsvorrichtungen für Gehörknöchelchen bekannt, die elastische Teile und/oder Gelenke aufweisen. Eine solche gelenkige Verbindung zwischen einem an der Steigbügelfußplatte montierbaren Befestigungselement und dem länglichen Schaft ist in der EP 1 181 907 B1 beschrieben und wird von der Anmelderin unter dem Markennamen "Ball-Joint" angeboten.

Eine weitere, verschiedentlich auftretende Komplikation entsteht durch eine Unterbelüftung des Mittelohrraumes und damit einher gehende akute oder chronische Entzündungen, Tumorbildungen, Verwachsungen im Trommelfellbereich sowie Versteifungen desselben. Beispielsweise bei einer Dysfunktion der Eustachischen Röhre kann es im Mittelohr zu einem Unterdruck kommen, der eine Auskrempung bzw. Ausstülpung (sog. Retraktion) des Trommelfells und in der Folge eine Verwachsung etwa mit dem Steigbügel bewirken kann. Um dem entgegen zu wirken und postoperativen Bewegungen des Trommelfells folgen zu können, werden die Kopfplatten bei bekannten Gehörknöchelchenprothesen verkippbar relativ zu dem Verbindungselement gestaltet, das die Kopfplatte mit dem zweiten Befestigungselement verbindet und meistens als länglicher Schaft ausgeführt ist. Eine solche in sich starre, aber gegenüber dem Verbindungselement verkippbare Kopfplatte ist u.a. beschrieben in der US 2004/0162614 A1, in dem Artikel M.W. YUNG, Ph.D., F.R.C.S., D.L.O., C. BREWIS, F.R.C.S., "A comparison of the user-friendliness of hydroxyapatite and titanium ossicular prostheses", The Journal of Laryngology & Otology, February 2002, Vol. 116, pp. 97-102, oder beispielsweise auch in der US 2006/0271190 A1.

Nachteilig bei diesen bekannten Gehörknöchelchenprothesen ist allerdings, dass durch die in sich starre Verkippung der Kopfplatte bei lokalen Medialbewegungen des Trommelfells gleichzeitig die gegenüber liegende Seite der Kopfplatte lateral nach außen bewegt wird, wodurch Druckspitzen auf das Trommelfell erzeugt werden.

Um eine hohe postoperative Flexibilität und Variabilität der Prothese zu erreichen, wobei gleichzeitig die Qualität der Schallleitung durch die Prothese erheblich erhöht werden soll, ohne dass es zu den oben geschilderten Komplikationen kommen kann, schlägt die eingangs zitierte DE 10 2007 013 708 B3 vor, dass die Stegelemente geometrisch so gestaltet sind, dass sie bei lokaler Medialbewegung des Trommelfells dieser Medialbewegung lokal folgen, jedoch die Bewegung nicht an entfernte Bereiche der Kopfplatte weitergeben. Durch diese flexible Gestaltung der Gehörknöchelchenprothese wird bei einer solchen kleineren Medialbewegung des Trommelfells eine starre Verkippung der gesamten Kopfplatte vermieden. Vielmehr verwindet sich die Kopfplatte lokal in sich selbst, wobei sie jedoch bei großflächigen, durch Schall verursachten Bewegungen des Trommelfells diese an das Verbindungselement weitergibt, so dass eine optimale Übertragung des Schalls bzw. des Schallsignals vom Trommelfell zum Mittelohrraum hin und weiter ans Innenohr gewährleistet wird.

Damit wird zwar gegenüber dem übrigen bekannten Stand der Technik eine ganz erhebliche Verbesserung erzielt. Leider bestehen jedoch noch weitere Probleme, die mit diesen Maßnahmen alleine nicht gelöst werden können:

Pathologie und Anatomie können im Rahmen einer Tympanoplastik im menschlichen Mittelohr ganz unterschiedliche, spezifisch vom individuellen Patienten abhängige Struktur-Rekonstruktionen notwendig machen. Je nach Ausmaß und Form von eventuell noch vorhandenen und vielleicht teilweise intakten Teilen der Mittelohranatomie, wie des Hammers (=Malleus), des Ambosses (=Incus), des Steigbügels. (=Stapes) oder des Trommelfells, werden entsprechend viele, zum Teil ganz erheblich in Form und Größe voneinander abweichende Geometrien der einzusetzenden Mittelohrprothesen benötigt.

Da vor Beginn einer Mittelohr-Operation nur sehr schwer oder gar nicht vorhergesagt werden kann, wie sich die spätere Rekonstruktion des Trommelfells und der Gehörknöchelchenkette im Verlauf der Operation darstellen wird (wenn überhaupt, dann nur grob, aber praktisch niemals genau), müssen derzeit für jede aktuell durchzuführende Operation immer sehr viele Mittelohrprothesen mit unterschiedlichen Geometrien, Formen und Größen bereitgehalten werden, damit der Chirurg intra-operativ stets aus einer vorrätigen Vielfalt heraus die jeweils am besten passende Prothese auswählen kann, die es ihm ermöglicht, speziell auf die jeweilige Gegebenheit einzugehen. Ansonsten kann es dazu kommen, dass keine optimale Versorgung gewährleistet ist.

Hinzu kommt, dass die genannten intraoperativen Anpassungsprobleme der Gehörknöchelchenprothese nicht nur im Anlagebereich des ersten Befestigungselements an das Trommelfell auftreten können, sondern ebenso im Bereich eines gegebenenfalls erforderlichen, ebenfalls plattenförmigen zweiten Befestigungselements, das zur Anlage der Prothese auf der Steigbügelfußplatte dienen kann. Insbesondere für den zum Innenohr hin abschließenden Bereich einer Totalrekonstruktion weist eine Totalprothese zu diesem Zweck normalerweise einen Stempel mit einem Standard-Durchmesser von 0,8 mm auf. Oftmals wird von chirurgischer Seite aus der Wunsch geäußert, dass man gerne - je nach intraoperativer Situation - unterschiedliche Größenareale zur Verfügung hätte, die auf die Fußplatte des Steigbügels aufgesetzt werden können. Die Bereitstellung eines zusätzlichen, mit dem Stempel verbundenen oder verbindbaren Befestigungselements, welches hinsichtlich der Größe seiner Fläche in weiten Grenzen variabel wäre, würde diesem Wunsch der Fachwelt entgegen kommen.

Falls die Gehörknöchelchenprothese keine Totalprothese und damit das erste Befestigungselement nicht als Kopfplatte zur Anlage am Trommelfell ausgebildet ist, sondern als Klammer zur Befestigung der Prothese an einem Glied der Gehörknöchelchenkette, treten die beschriebenen Anpassungsprobleme eventuell auch ausschließlich am Innenohrseitigen Ende der Gehörknöchelchenprothese auf.

Ein weiteres Problem liegt darin, dass - Weltweit gesehen - äußerst unterschiedliche chirurgische Techniken angewendet werden, welche verschiedenartige Rekonstruktionen im Mittelohr postulieren. Diese erfordern entsprechend angepasste, voneinander in Form und Größe sehr unterschiedliche Mittelohrprothesen, welche wiederum während jeder Operation vorrätig gehalten werden müssen, um dem Chirurgen die Möglichkeit zur geben, die nach seiner Einschätzung jeweils für den aktuell vorgefundenen Fall optimale Methode anzuwenden.

Aufgabe der vorliegenden Erfindung ist es demgegenüber, eine gattungsgemäße Mittelohrprothese der eingangs beschriebenen Art mit möglichst einfachen technischen Mitteln unaufwändig und kostengünstig dahin gehend zu verbessern, dass die Anzahl der intraoperativ bereit zuhaltenden unterschiedlichen Prothesen ganz erheblich verringert, vorzugsweise auf eine einzige Standard-Prothese reduziert werden kann, ohne dabei die Möglichkeit zur optimalen Adaption der Prothese im konkreten Einzelfall zu verlieren.

Erfindungsgemäß wird diese Aufgabe auf ebenso überraschend einfache wie wirkungsvolle Art und Weise dadurch gelöst, dass der Ankoppelbereich, die Stegelemente und die radial äußeren Abschnitte geometrisch derart gestaltet sind und ihr Material so gewählt ist, dass durch Auseinanderziehen bzw. Zusammenstauchen von Ankoppelbereich, Stegelementen und radial äußeren Abschnitten in der Plattenebene des plattenförmigen ersten Befestigungselements eine bleibende plastische Verformung erfolgt, durch die der Außendurchmesser des ersten Befestigungselements in diesem Bereich dauerhaft vergrößert bzw. verkleinert wird, und dass einander in der Ringebene gegenüber liegende radial äußere Abschnitte des plattenförmigen ersten Befestigungselements jeweils einen die Ringebene des äußeren Ringbereichs durchstoßenden, in der Ringebene in sich geschlossenen Durchbruch aufweisen.

In diese Durchbrüche, als runde Bohrungen oder in anderen geometrischen Formen ausgeführt sein können, hakt der Operateur während der Implantation der Prothese jeweils ein hakenförmiges Ende eines geeigneten chirurgischen Instruments ein und zieht dann die entsprechenden radial äußeren Abschnitte der Kopfplatte auseinander oder staucht sie gegeneinander zusammen. Vergleichbares ist im bekannten Stand der Technik weder zu finden noch wäre es dort sinnvoll verwendbar.

Plastisch dehnbare bzw. stauchbare sowie auch verbiegbare Bereiche innerhalb des plattenförmigen ersten Befestigungselements haben - wenn die Form und Größe der Platte nach der Dehnung, Stauchung oder Verbiegung dauerhaft erhalten bleibt - den Vorteil, dass man der Platte je nach Bedarf erheblich mehr Stabilität "einimpfen" kann. So können beispielsweise die Stege des ersten Befestigungselements, wenn man sie ganz nach außen dehnt, als Stabilisatoren umfunktioniert werden. Weiter können die Stege aber auch in Richtung der Längsachse der Gehörknöchelchenprothese gebogen werden und so der neuen Rekonstruktion mehr Stabilität verleihen.

Das plattenförmige erste Befestigungselement der erfindungsgemäßen Mittelohrprothese, welches z.B. als Kopfplatte ausgestaltet sein kann, die im Rahmen einer Tympanoplastik gegen das Trommelfell gelegt wird, ist so aufgebaut, dass es in seiner Form und Fläche in sehr weiten Grenzen variabel ist. Die standardmäßig vorrätig gehaltene erfindungsgemäße Prothese lässt sich daher intraoperativ in allen Belangen ganz einfach, sehr variabel und äußerst zielgenau so umgestalten, wie es die vorgefundene patientenspezifische Situation jeweils gerade erfordert.

Einfache, ad hoc vornehmbare Veränderungen der erfindungsgemäßen Standard-Prothese, etwa hinsichtlich von Winkeln, Längen oder Flächengrößen, dienen einer erheblich verbesserten Adaption im konkreten Einzelfall. Somit bietet die erfindungsgemäße MittelohrProthese dem Operateur ein extrem hohes Maß an Variabilität und Flexibilität, ohne dass dazu die bisher übliche Bevorratung einer großen Vielzahl unterschiedlichster Prothesenformen, -größen und -geometrien erforderlich ist. Der Chirurg kann damit intraoperativ an der Prothese gezielte Veränderungen vornehmen, die es ihm ermöglichen, die Prothese speziell auf die jeweilige Gegebenheit einzustellen bzw. anzupassen.

Durch die erfindungsgemäß angewandte Technik einer dauerhaften plastischen Verformung ist es ganz leicht möglich, beispielsweise die Kopfplatte der Prothese in ihrer Größe und Form so zu verändern, dass sie nur speziell gewollte Bereiche am Trommelfell berührt; in der Regel diejenigen, bei denen man genau weiß, dass sie für die akustische Übertragung eine wesentliche Rolle spielen.

Ähnliches gilt auch für eine Ankopplung der Gehörknöchelchenprothese an die Steigbügelfußplatte, wo ebenfalls durch die erfindungsgemäße flächenvariable Gestaltung des entsprechenden plattenförmigen Befestigungselements eine bisher nicht gekannte intraoperative Flexibilität im Hinblick auf eine optimale Anpassung an die individuelle Patientensituation geboten werden kann.

Von der erfindungsgemäßen Grundidee kann in doppelter Weise profitiert werden, wenn beide Befestigungselemente plattenförmig aufgebaut und plastisch dauerhaft verformbar gestaltet sind, wobei das erste Befestigungselement zur Anlage der Gehörknöchelchenprothese auf der Steigbügelfußplatte ausgebildet ist und das zweite Befestigungselement als ebene Kopfplatte zur mechanischen Verbindung mit dem Trommelfell dient.

Besonders günstig ist es, wenn die Stegelemente eine maximale Breite b und der radial äußere Ringbereich eine maximale Breite B aufweisen, wobei gilt: 2b < B.

Um die gewünschte Flexibilität zu erreichen, sollte bei einer maximalen Breite b der Stegelement und einem minimalen Durchmesser D des plattenförmigen ersten Befestigungselements einschließlich des Ringbereiches gelten: b ≤ 0,05D, vorzugsweise b ≈ 0,03D. Beim Stand der Technik, etwa bei den in der US 2004/0162614 A1 beschriebenen Gehörknöchelchenprothesen, liegt das Verhältnis b/D mindestens bei 0,1 oder darüber.

Weiter ist es günstig, wenn das plattenförmige erste Befestigungselement der erfindungsgemäßen Gehörknöchelchenprothese eine Dicke, insbesondere eine Blechdicke t zwischen 0,01mm und 0,5mm, vorzugsweise zwischen 0,1mm und 0,25mm, sowie einen minimalen Durchmesser D zwischen 1,5mm und 8mm, vorzugsweise zwischen 2mm und 5mm, und die Stegelemente eine maximale Breite b zwischen 0,01mm und 0,3mm, vorzugsweise zwischen 0,05mm und 0,2mm aufweisen.

Durch die Unterbrechungen im radial äußeren Ringbereich lassen sich asymmetrische Formgestaltungen leicht bewerkstelligen. Vor allem aber wird die plastische Verformung des plattenförmigen ersten Befestigungselements durch Ziehen oder Stauchen des in Abschnitte geteilten Ringbereichs innerhalb der Plattenebene erleichtert. Der radial äußere Ringbereich kann bei Ausführungsformen eine ovale oder kreisrunde Form aufweisen, was aus dem Stand der Technik an sich bekannt und einfach herstellbar ist. Dies wird in der Regel die Standard-Variante für die erfindungsgemäße Gehörknöchelchenprothese bilden.

Zur Erleichterung des operativen Einsetzens der erfindungsgemäßen Gehörknöchelchenprothese kann bei einer speziellen Variante der radial äußere Ringbereich eine einseitige Einbuchtung zur Aufnahme des Hammergriffs aufweisen.

Eine weitere Variante sieht vor, dass der radial äußere Ringbereich des plattenförmigen ersten Befestigungselements in der Plattenebene eine radial nach außen verlaufende Ausbuchtung aufweist, die in Strukturen der Gehörknöchelchenkette eingreifen kann.

Möglich ist aber auch eine Variante, bei der der radial äußere Ringbereich eine Schlangenlinienförmige Außenkontur aufweist, die sich in speziellen geometrischen Mittelohrsituationen, wie sie beim Patienten in der Praxis oftmals vorgefunden werden können, als günstig erweisen kann.

Eine weitere vorteilhafte Ausführungsform der erfindungsgemäßen Gehörknöchelchenprothese zeichnet sich dadurch aus, dass die Stegelemente in der Plattenebene des plattenförmigen ersten Befestigungselements nicht geradlinig, sondern auf - insbesondere mehrfach - gekrümmten Kurven verlaufen, wodurch sich die erwünschte Wirkung einer lokalen Flexibilität vor allem im Falle einer Trommelfell-Kopfplatte und eines lediglich lokal begrenzten Ausweichens bei kleineren Medialbewegungen des Trommelfells ergibt. Außerdem kann dadurch eine Kopfplatte einer eventuellen post-operativen Veränderung des Trommelfells leichter folgen.

Bei weiteren vorteilhaften Ausführungsformen der erfindungsgemäßen Gehörknöchelchenprothese ist jedes Stegelement mit mindestens zwei anderen Stegelementen verbunden, so dass eine Art flexibles Netzwerk aus Stegelementen entsteht.

Bevorzugt ist auch eine Klasse von Ausführungsformen der Erfindung, bei denen mindestens ein Verlängerungsteil vorgesehen ist, das wie ein Puzzle-Teil am Außenrand des plattenförmigen ersten Befestigungselements in der Plattenebene von der Seite her angeknöpft werden kann. Dadurch eröffnen sich hinsichtlich der geometrischen Form des Befestigungselements enorm viele Gestaltungsmöglichkeiten. Insbesondere müssen jetzt nicht mehr unbedingt symmetrische Formen implantiert werden, wie sie bisher durch die vorhandenen Standard-Prothesen zwangsläufig vorgegeben waren. Vielmehr kann der Operateur ganz leicht und noch während der Operation eine handgefertigte, optimal angepasste Gehörknöchelchenprothese zielgenau "tailor-made" herstellen.

Oftmals liegt der Fall vor, dass der Hammergriff am Trommelfell vorhanden ist - oder eben auch fehlt - je nach dem wie oft voroperiert, welche Operationsmethode angewendet und welche spezifische Maßnahme ergriffen wurde. Entsprechend kann bei einer erfindungsgemäß flächenvariabel gestalteten Kopfplatte durch den Operateur zielgenau und exakt richtig reagiert werden, also der für den Hammergriff verantwortliche Bereich abgenommen oder - nach Bedarf - angefügt werden. Bei vorteilhaften Weiterbildungen dieser Klasse von Ausführungsformen ist dazu das seitlich anknöpfbare Verlängerungsteil als Appendix für den Hammer oder den Hammergriff der Gehörknöchelchenprothese geformt und ragt im zusammengeknöpften Zustand spitz vom Rand des plattenförmigen ersten Befestigungselements radial nach außen weg.

Diese Weiterbildungen lassen sich noch dadurch verbessern, dass das seitlich anknöpfbare Verlängerungsteil federnd im ersten Befestigungselement verankert ist, was insbesondere ein unbeabsichtigtes Abbrechen des sehr feinen Miniaturteils beim Anknöpfen in das Befestigungselement erschwert.

Die federnde Verankerung des Verlängerungsteils lässt sich beispielsweise mittels eines einfachen Klammerelements erreichen.

Bei weiteren vorteilhaften Varianten ist das seitlich anknöpfbare Verlängerungsteil einrastend und damit verliersicher im ersten Befestigungselement verankert, insbesondere mittels Widerhaken.

In der Regel wird bei der erfindungsgemäßen GehörknöchelchenProthese das Verbindungselement zwischen den Befestigungselementen als länglicher Schaft ausgeführt, wie dies an sich aus dem Stand der Technik wohlbekannt ist.

Um die oben erörterte Flexibilität bzw. Variabilität der Prothese - wie sie an sich in der EP 1 181 907 B1 beschrieben ist - zu erhöhen, kann bei einer besonders bevorzugten Weiterbildung dieser Ausführungsform am oder im länglichen Schaft mindestens ein Kugelgelenk vorgesehen sein. Vorteilhaft im Hinblick auf eine besonders hohe postoperative Beweglichkeit der Prothese sind Varianten, bei denen der längliche Schaft eine Vielzahl von aneinander angrenzenden weiteren Drehelementen, vorzugsweise eine Kugelgelenkkette, umfasst.

Alternativ kann der Schaft bei besonders einfachen und preisgünstig herstellbaren Ausführungsformen der erfindungsgemäßen Prothese aber auch einstückig durchgehend, insbesondere starr ausgeführt sein.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. In vielen in der Praxis eingesetzten Ausführungsformen der Erfindung wird das erste Befestigungselement eine zur Anlage am Trommelfell ausgebildete Kopfplatte umfassen. Bei vielen weiteren Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine. Klasse von Ausführungsformen der erfindungsgemäßen Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke, als Stempel oder als Clip zur mechanischen Verbindung mit einem weiteren Glied der Gehörknöchelchenkette ausgebildet ist.

Bei Weiterbildungen dieser Ausführungsformen ist die Prothese über das als Kopfplatte ausgebildete erste Befestigungselement einerseits am Trommelfell und über das zweite Befestigungselement andererseits am Amboss oder am Steigbügel befestigt.

Alternative Ausgestaltungen können vorsehen, dass die Gehörknöchelchenprothese mittels Perforation der Steigbügelfußplatte (=Stapedektomie bzw. Stapedotomie) und/oder mittels Eröffnung der menschlichen Hörschnecke (=Cochleotomie) an dem der Trommelfellseite entgegen gesetzten anderen Ende direkt an das Innenohr angekoppelt ist, insbesondere über einen Kolben.

Möglich sind Ausführungsformen der Erfindung, bei denen die Prothese oder Teile davon aus biokompatiblen Kunststoffen, insbesondere Silikon, Polytetrafluorethylen (PTFE) oder Polyetheretherketon (PEEK), und/oder aus Faserverbundwerkstoffen, insbesondere Kohlefasern hergestellt sind. Mit diesen Materialien können postoperative Abstoßungsreaktionen in den meisten Fällen verhindert werden.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Ein als Kopfplatte ausgebildetes Befestigungselement sollte bei der erfindungsgemäßen Gehörknöchelchenprothese grundsätzlich eine wachstumsfördernde Beschichtung, ein direkt ins Innenohr führendes, etwa in Form eines Kolbens ausgebildetes Befestigungselement hingegen eine wachstumshemmende Beschichtung aufweisen.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgegebenen oder vorgebbaren Frequenzgang der Schallleitung im Mittelohr berechnet ist. Damit lässt sich ohne großen zusätzlichen technischen Aufwand gewissermaßen ein mechanisches Tuning der Schallfortpflanzungseigenschaften mittels einer individuellen ausgestalteten Gehör-knöchelchenprothese erreichen.

Ein solcher Tuning-Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Eine weitere Ausführungsform der Erfindung schließlich zeichnet sich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Gehörknöchelchenprothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

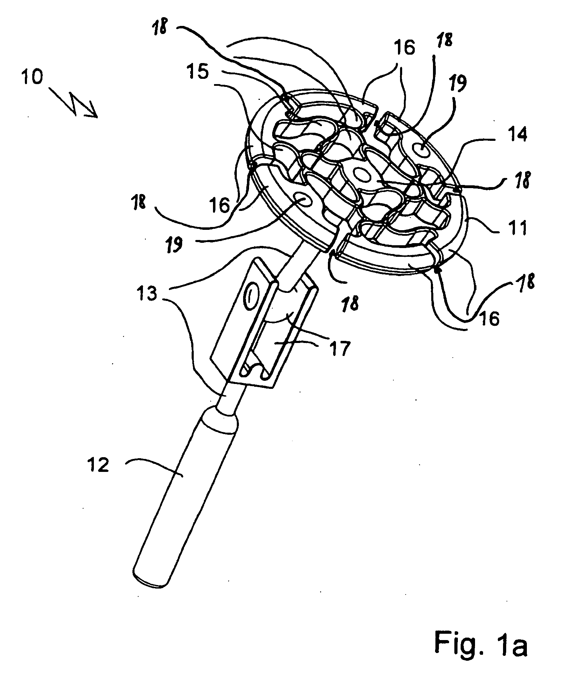

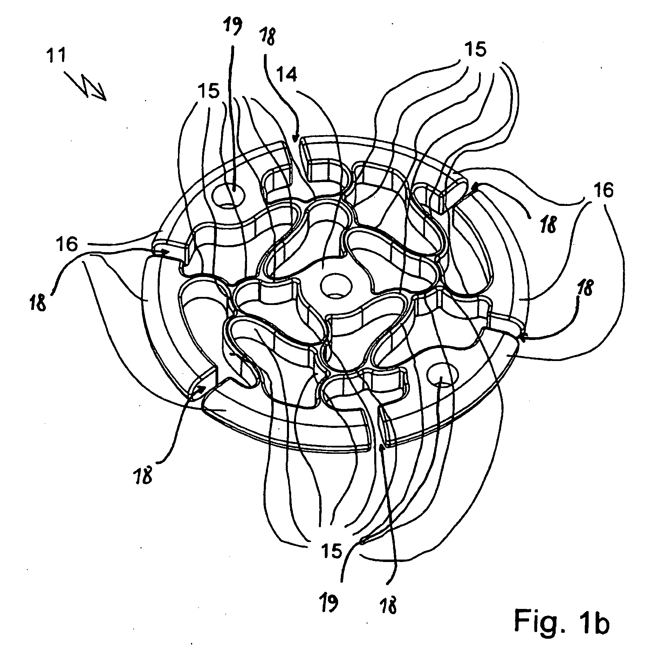

Die Figuren 1a-b, 2a-b und 3a-c bis 7a-c der Zeichnung sind in entweder in 2er- oder in 3er-Gruppen unterteilt, wobei die jeweiligen Einzelfiguren einer Gruppe voneinander durch eine Nummerierung mit a, b und gegebenenfalls c unterschieden sind. Die "a"-Figuren enthalten jeweils eine schematische räumliche Darstellung einer Ausführungsform der erfindungsgemäßen Gehörknöchelchenprothese, die "b"-Figuren das zur entsprechenden "a"-Figur gehörige, erfindungsgemäß ausgestaltete erste Befestigungselement und die "c"-Figuren ggf. das erste Befestigungselement der entsprechenden "b"-Figur mit einem seitlich anknöpfbaren Verlängerungsteil im auseinander geknöpften Zustand. Im Übrigen sind in der Zeichnung Elemente mit gleichem Aufbau und/oder gleicher Funktion mit der derselben Bezugsziffer gekennzeichnet.

Im Einzelnen zeigen:
- Fign. 1a-b: eine Ausführungsform der erfindungsgemäßen Gehörknöchelchenprothese mit einem als Trommelfell-Kopfplatte gestalteten, ringförmig aufgebauten ersten Befestigungselement einem Kugelgelenk im Verbindungselement und einem kolbenförmigen zweiten Befestigungselement;
- Fign. 2a-b: eine Ausführungsform mit zwei plattenförmig aufgebauten Befestigungselementen;
- Fign. 3a-c: eine Ausführungsform mit einem seitlich anknöpfbaren, als Appendix für den Hammer oder den Hammergriff geformten Verlängerungsteil am ersten Befestigungselement sowie einem klammerförmigen zweiten Befestigungselement;
- Fign. 4a-c: eine Ausführungsform mit seitlich anknöpfbarem Verlängerungsteil und radial nach außen verlaufender Ausbuchtung des ersten Befestigungselements nach dem Abknöpfen des anknöpfbaren Verlängerungsteil sowie einer geschlitzten Glocke als zweitem Befestigungselement;
- Fign. 5a-c: eine Ausführungsform mit einem federnd im ersten Befestigungselement verankerten, seitlich anknöpfbaren Verlängerungsteil;
- Fign. 6a-c: eine Ausführungsform mit einem mittels eines Klammerelements im ersten Befestigungselement verankerten seitlich anknöpfbaren Verlängerungsteil; und
- Fign. 7a-c: eine Ausführungsform mit einem einrastend im ersten Befestigungselement verankerten, seitlich anknöpfbaren Verlängerungsteil.

Die erfindungsgemäßen **Gehörknöchelchenprothesen 10; 20; 30; 40; 50; 60; 70** weisen jeweils an einem Ende ein plattenförmiges **erstes Befestigungselement 11; 21; 31; 41; 51; 61; 71** auf, welches in Form einer Kopfplatte zur Anlage am Trommelfell oder als Fußplatte zur Anlage an der Steigbügelfußplatte ausgebildet ist. Am anderen Ende der Gehörknöchelchenprothesen 10; 20; 30; 40; 50; 60; 70 sitzt jeweils ein **zweites Befestigungselement 12; 22; 32; 42** zur mechanischen Verbindung der Prothese mit einem Glied oder Teilen eines Gliedes der Gehörknöchelchen-Kette oder direkt mit dem Innenohr. Dazwischen ist ein die beiden Befestigungselemente Schall leitend miteinander verbindendes **Verbindungselement 13; 23; 33** angeordnet, welches bei den gezeigten Ausführungsformen in Form eines ein- oder mehrteiligen, kurzen oder längeren Schaftes ausgeführt ist.

Das plattenförmige erste Befestigungselement 11; 21; 31; 41; 51; 61; 71 weist jeweils einen radial inneren, insbesondere um seinen Flächenschwerpunkt zentral angeordneten **Ankoppelbereich 14; 24; 34; 44; 54; 64; 74** zum mechanischen Ankoppeln des ersten Befestigungselements an das Verbindungselement 13; 23; 33 sowie mehrere **Stegelemente 15; 25,25',25"; 35,35',35",35"'; 45,45'; 55,55', 55"; 65,65'; 75,75',75"** zur radialen Verbindung des radial inneren Ankoppelbereichs mit **radial äußeren Abschnitten 16; 26; 36,36'; 46,46'; 56,56'; 66,66'; 76,76'** des ersten Befestigungselements 11; 21; 31; 41; 51; 61; 71 auf.

Der Ankoppelbereich 14; 24; 34; 44; 54; 64; 74 und/oder die Stegelemente 15; 25,25',25"; 35,35',35",35"'; 45,45'; 55,55', 55"; 65,65'; 75,75',75" und/oder die radial äußeren Abschnitte 16; 26; 36,36'; 46,46'; 56,56'; 66,66'; 76,76' sind geometrisch so gestaltet und ihr Material so gewählt, dass durch Auseinanderziehen bzw. Zusammenstauchen von Ankoppelbereich 14; 24; 34; 44; 54; 64; 74, Stegelementen 15; 25,25',25"; 35,35',35",35"'; 45,45'; 55,55',55"; 65,65'; 75,75',75" und radial äußeren Abschnitten 16; 26; 36,36'; 46,46'; 56,56'; 66,66'; 76,76' in der Plattenebene des plattenförmigen ersten Befestigungselements 11; 21; 31; 41; 51; 61; 71 eine dauerhafte plastische Verformung erfolgt, durch die der Außendurchmesser des ersten Befestigungselements 11; 21; 31; 41; 51; 61; 71 in diesem Bereich dauerhaft vergrößert bzw. verkleinert wird.

Die radial äußeren Abschnitte 16; 26; 36,36'; 46,46'; 56,56'; 66,66'; 76,76' des plattenförmigen ersten Befestigungselements 11; 21; 31; 41; 51; 61; 71 bilden einen äußeren Ringbereich mit mindestens einer, vorzugsweise mehreren **Unterbrechungen 18; 28; 38; 48; 58; 68; 78.** Einander in der Ringebene gegenüber liegende radial äußere Abschnitte 16; 26; 36,36'; 46,46'; 56,56'; 66,66'; 76,76' des plattenförmigen ersten Befestigungselements 11; 21; 31; 41; 51; 61; 71 weisen jeweils einen die Ringebene des äußeren Ringbereichs durchstoßenden, in der Ringebene in sich geschlossenen **Durchbruch 19; 29; 39; 49; 59; 69; 79** auf, der als runde Bohrung, als ovales Loch oder in anderer geometrischer Ausgestaltung ausgeführt sein kann.

Die in den Figuren 1a-b dargestellte Ausführungsform weist als erstes Befestigungselement 11 eine Kopfplatte und als Verbindungselement 13 einen zweigeteilten Schaft auf, der zwischen seinen beiden Teilen ein **Kugelgelenk 17** enthält, um die mechanische Flexibilität des Schaftes zu erhöhen. Das zweite Befestigungselement 12 an dem der Kopfplatte entgegen gesetzten Ende der in Fig. 1a dargestellten Gehörknöchelchenprothese 10 ist im vorliegenden Ausführungsbeispiel als Kolben zur direkten Ankopplung der Gehörknöchelchenprothese 10 an das Innenohr ausgebildet.

Das erste Befestigungselement 11 weist Durchbrüche durch die Plattenebene zur Erhöhung der Flexibilität der Kopfplatte bei Verwindungen in und aus der Plattenebene auf und umfasst einen radial inneren angeordneten Ankoppelbereich 14. Weiteres, radial äußere Abschnitte 16 umgeben den radial inneren Ankoppelbereich 14 ringförmig, wobei der radial äußere Ringbereich eine im Wesentlichen kreisrunde Form hat. Die radial äußeren Abschnitte 16 sind mit dem radial inneren Ankoppelbereich 14 über eine Vielzahl von mehrfach verzweigten, auf gekrümmten Kurven verlaufenden dünnen Stegelementen 15, die ein flexibles Netzwerk bilden, verbunden.

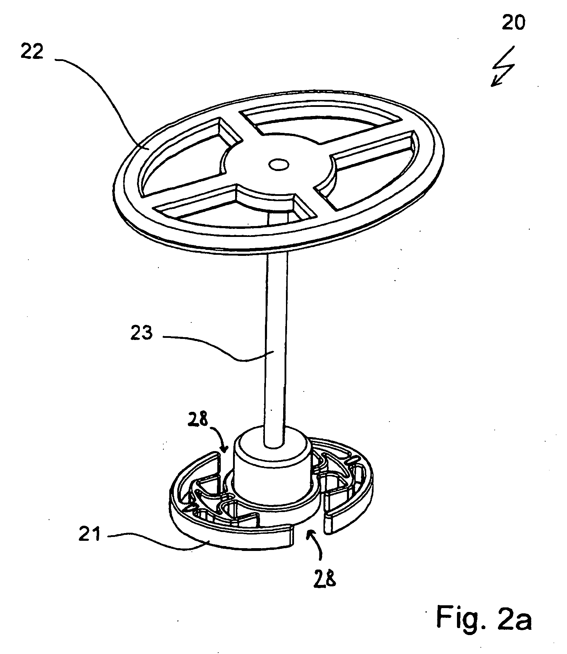

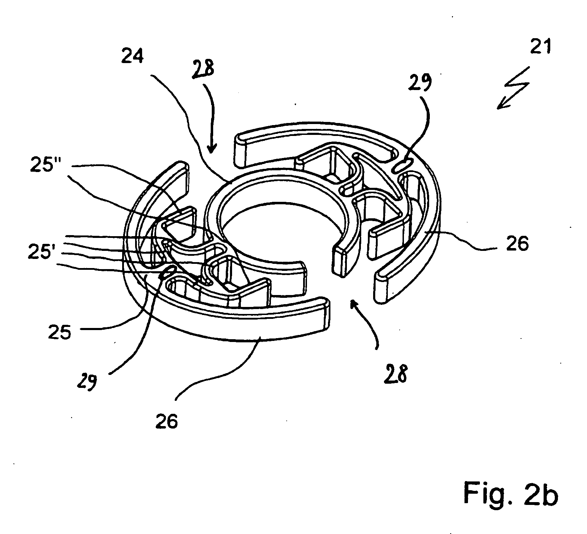

Bei der Ausführungsform nach den Figuren 2a-b sind alle beide Befestigungselemente 21, 22 plattenförmig gestaltet und erfindungsgemäß wiederum plastisch verformbar, wobei das erste Befestigungselement 21 zur Anlage der Gehörknöchelchenprothese 20 auf der Steigbügelfußplatte ausgebildet ist, während das zweite Befestigungselement 22 als ebene Kopfplatte zur mechanischen Verbindung mit dem Trommelfell dient. Da es sich hier um eine Totalprothese handelt, ist das Verbindungselement 23 als einstückig durchgehender, starrer langer Schaft ausgeführt. Auch beim ersten Befestigungselement 21 wird ein radial innerer Ankoppelbereich 24 über eine Vielzahl von verzweigten Stegelementen 25, 25', 25" mit radial äußeren Abschnitten 26 verbunden, die den Ankoppelbereich 24 ringförmig umgeben, wobei auch der radial innere Ankoppelbereich 24 als Ringbereich mit einer Unterbrechung in seiner Azimutalebene gestaltet ist. Wie in Fig. 2a gezeigt ist, kann der Ankoppelbereich 24 beispielsweise leicht auf eine kolbenförmige Verdickung des Verbindungselements 23 an seinem dem zweiten Befestigungselement 22 abgewandten Ende geklemmt werden.

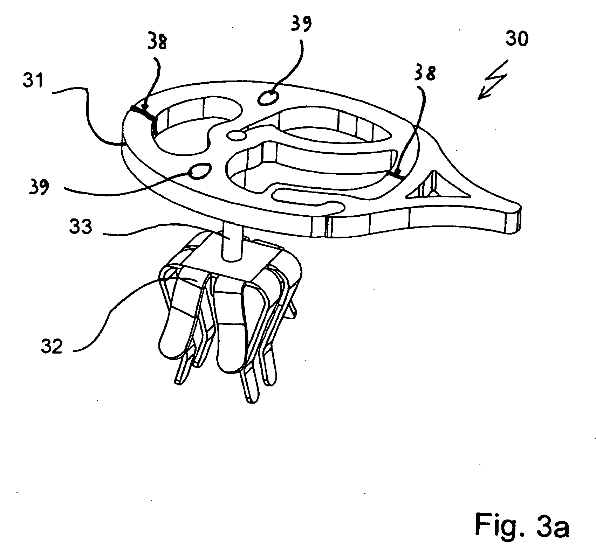

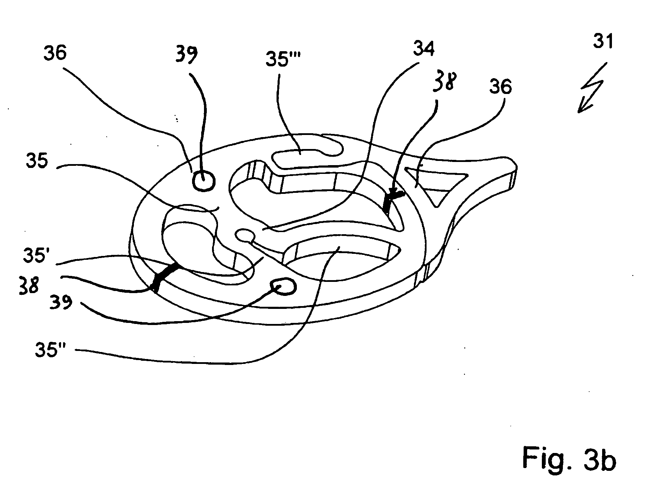

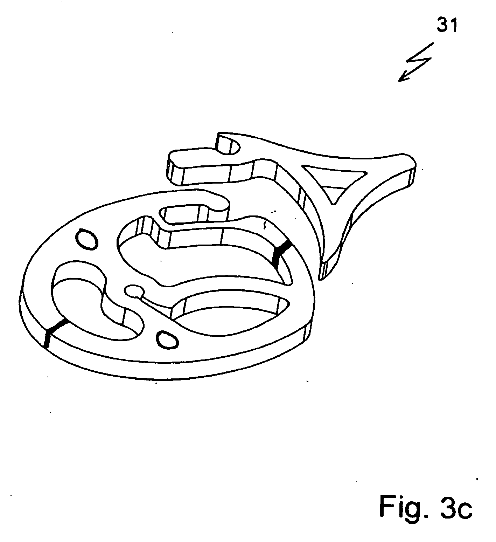

Die Ausführungsformen der Figuren 3a-c bis 7a-c zeichnen sich dadurch aus, dass das erste Befestigungselement 31; 41; 51; 62, 71 jeweils ein Verlängerungsteil 36'; 46'; 56'; 66'; 76' umfasst, das wie ein Puzzle-Teil am Außenrand des plattenförmigen ersten Befestigungselements 31; 41; 51; 61; 71 in der Plattenebene von der Seite her an den radial äußeren Abschnitt 36; 46; 56; 66; 76 angeknöpft werden kann. Bei den in der Zeichnung dargestellten Ausführungsformen ist dieses seitlich anknöpfbare Verlängerungsteil 36'; 46'; 56'; 66'; 76' jeweils als Appendix für den Hammer oder den Hammergriff der Gehörknöchelchenprothese 30; 40; 50; 60; 70 geformt und ragt im zusammengeknöpften Zustand spitz vom Rand des plattenförmigen ersten Befestigungselements 31; 41; 51; 61; 71 radial nach außen weg.

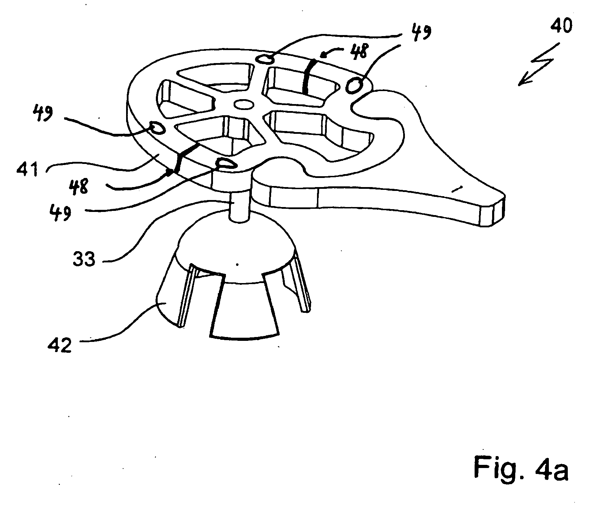

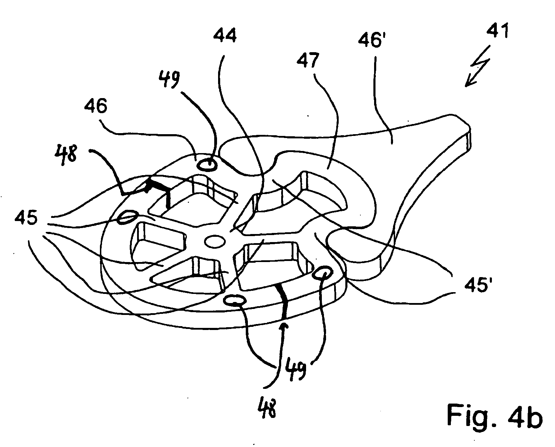

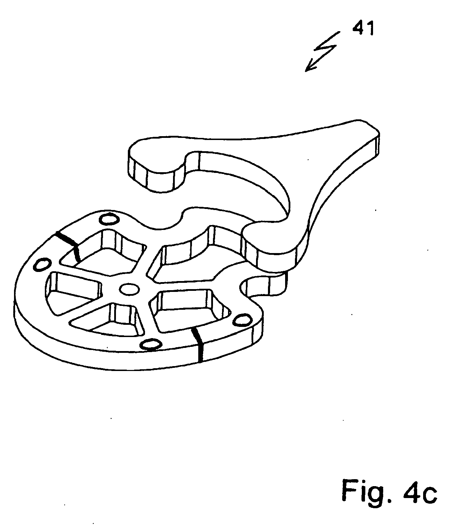

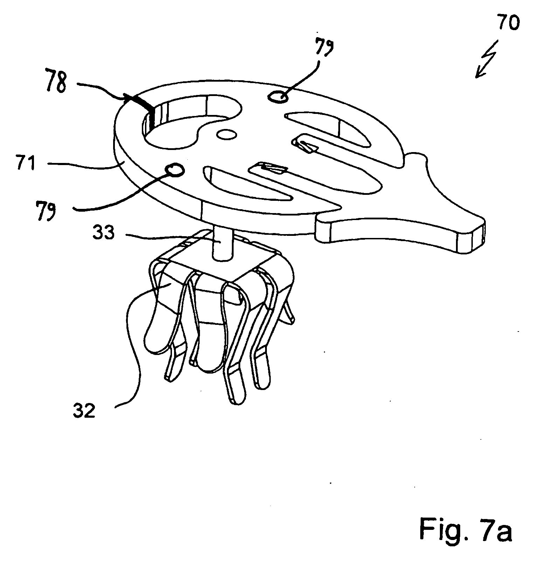

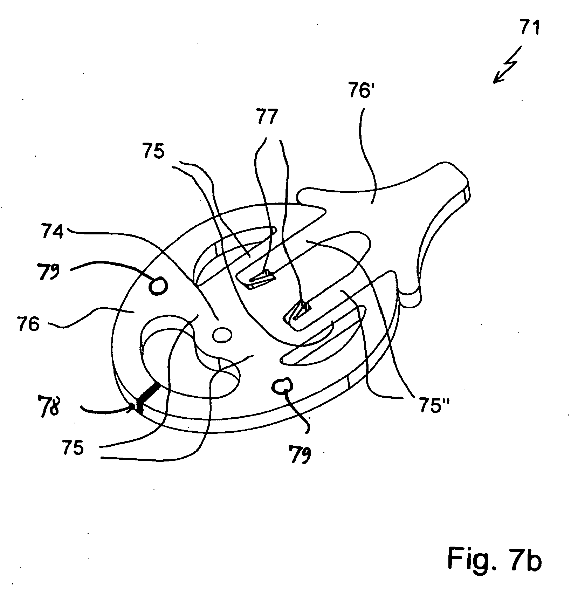

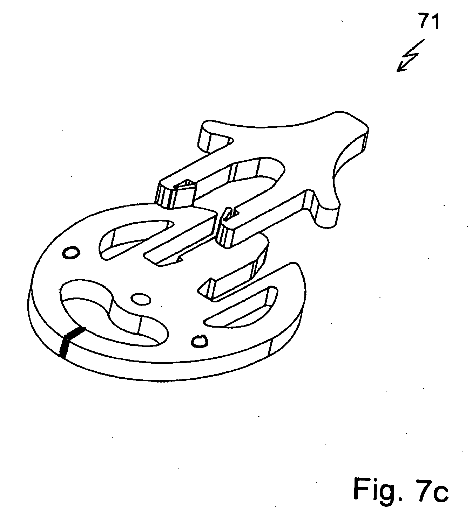

Die zweiten Befestigungselemente 32; 72 bei den Gehörknöchelchenprothesen 30; 70 nach den Figuren 3a und 7a sind jeweils als Klammer mit mehreren Zungen ausgeführt, während sie bei den Ausführungsformen nach den Figuren 4a bis 6a jeweils eine geschlitzte Glockenform aufweisen. Beide Gestaltungen dienen zur Befestigung der jeweiligen Gehörknöchelchenprothese 30; 40; 50; 60; 70 an einem Glied der Gehörknöchelchenkette, beispielsweise am Amboss oder am Steigbügel.

Das plattenförmige erste Befestigungselement 41 der Ausführungsform nach den Figuren 4a-c zeichnet sich dadurch aus, dass sein radial äußerer Abschnitt 46 eine in der Plattenebene radial nach außen verlaufende **Ausbuchtung 47** aufweist, die auch schon ohne das zweite, seitlich anknöpfbare Verlängerungsteil 46' als Appendix für den Hammer oder den Hammergriff der Gehörknöchelchenprothese 40 dienen kann, falls eine kleinere Gesamtfläche des ersten Befestigungselements 41 gewünscht wird.

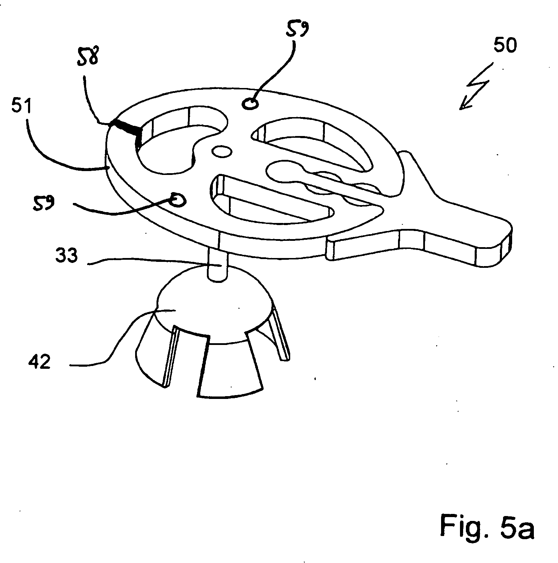

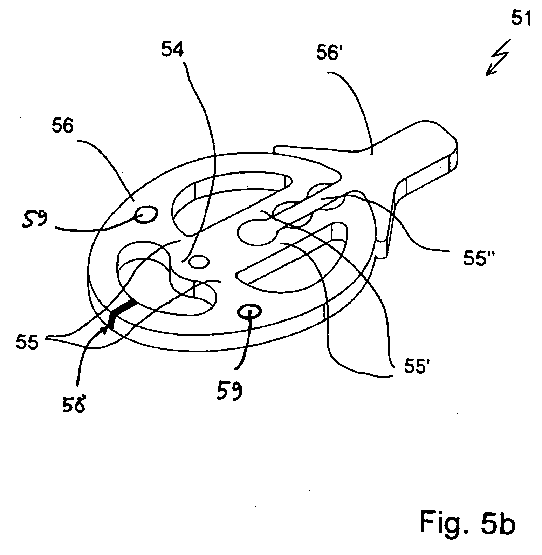

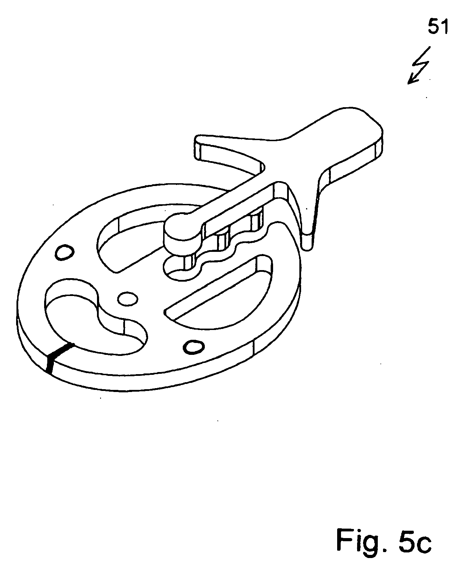

Bei den ersten Befestigungselementen 51; 61; 71 der Gehörknöchelchenprothesen 50; 60; 70 gemäß den Figuren 5a-c bis 7a-c ist jeweils das seitlich anknöpfbare Verlängerungsteil 56'; 66'; 76' federnd im radial äußeren Abschnitt 56; 66; 76 des ersten Befestigungselements 51; 61; 71 verankert.

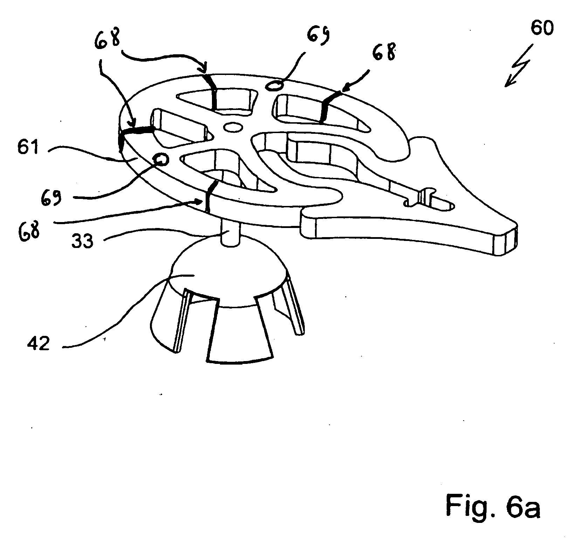

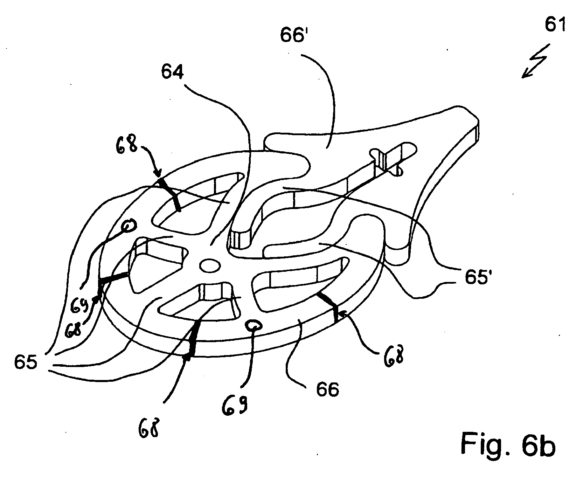

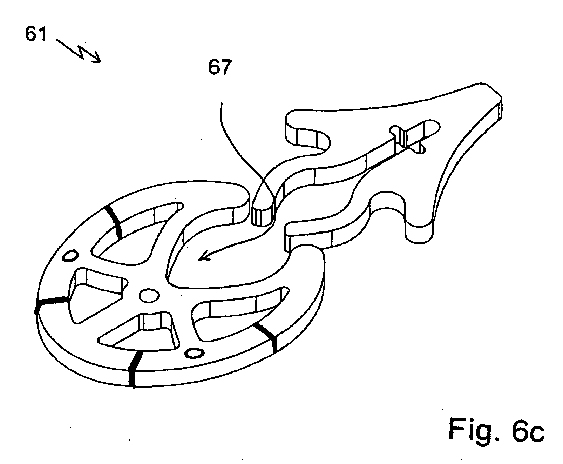

Die anknöpfbaren Verlängerungsteile 66'; 76' der Ausführungsformen nach den Figuren 6a-c bis 7a-c können jeweils mittels eines als Klammerelement ausgebildeten Paares von Stegelementen 65' bzw. 75" im jeweiligen radial äußeren Abschnitt 66 bzw. 76 des ersten Befestigungselements 61; 71 seitlich verankert werden.

Der radial äußere Ringbereich des ersten Befestigungselements 61 in der Ausführungsform nach den Figuren 6a-c weist eine einseitige **Einbuchtung 67** auf, die insbesondere zur Aufnahme des Hammergriffs dienen kann, wenn das anknöpfbare Verlängerungsteil 66' nicht verwendet wird.

Bei der Gehörknöchelchenprothese 70 nach den Figuren 7a-c schließlich wird das seitlich anknöpfbare Verlängerungsteil 76' mittels an den Enden der beiden Stegelemente 75" vorgesehener **Widerhaken 77** einrastend und damit verliersicher im ersten Befestigungselement 71 verankert.

Die Massenverteilung der einzelnen Teile der erfindungsgemäßen Gehörknöchelchenprothese 10; 20; 30; 40; 50; 60; 70 kann in Abhängigkeit von einem gewünschten, vorgebbaren Frequenzgang der Schallleitung im Mittelohr berechnet sein, um ein individuelles Tuning der Schallleitungs-Eigenschaften zu ermöglichen.

Bei in der Zeichnung nicht eigens dargestellten weiteren Ausführungsformen der erfindungsgemäßen Gehörknöchelchenprothese können die zentralen Ankoppelbereiche und/oder die Stegelemente und/oder die radial äußeren Abschnitte auch andere Geometrien besitzen, um die gewünschte Flächenvariabilität des jeweiligen ersten Befestigungselements zu erzielen. So können etwa zumindest einige Stegelemente eine Unterbrechung zwischen dem zentralen Ankoppelbereich und den radial äußeren Abschnitten aufweisen. Auch können die radial äußeren Abschnitte einfach oder mehrfach unterbrochen gestaltet sein, eine Schlangenlinienförmige Außenkontur und/oder eine einseitige Einbuchtung zur Aufnahme des Hammergriffs aufweisen.

## Patentansprüche

1. Gehörknöchelchenprothese (10; 20; 30; 40; 50; 60; 70), die mindestens ein Glied oder Teile eines Gliedes der Gehörknöchelchenkette ersetzt oder überbrückt, wobei die Gehörknöchelchenprothese (10; 20; 30; 40; 50; 60; 70) an ihrem einen Ende ein im Wesentlichen plattenförmiges erstes Befestigungselement (11; 21; 31; 41; 51; 61; 71) zur Anlage am Trommelfell oder an der Steigbügelfußplatte und an ihrem anderen Ende ein zweites Befestigungselement (12; 22; 32; 42) zur mechanischen Verbindung mit einem Glied oder Teilen eines Gliedes der Gehörknöchelchenkette oder mit dem Innenohr sowie ein die beiden Befestigungselemente (11,12; 21,22; 31,32; 41,42; 51,42; 61,42; 71,32) Schall leitend miteinander verbindendes Verbindungselement (13; 23; 33) umfasst, und wobei das plattenförmige erste Befestigungselement (11; 21; 31; 41; 51; 61; 71) einen radial inneren, insbesondere um den Flächenschwerpunkt des plattenförmigen ersten Befestigungselements (11; 21; 31; 41; 51; 61; 71) zentral angeordneten Ankoppelbereich (14; 24; 34; 44; 54; 64; 74) zum mechanischen Ankoppeln des ersten Befestigungselements (11; 21; 31; 41; 51; 61; 71) an das Verbindungselement (13; 23; 33) sowie mehrere Stegelemente (15; 25,25',25"; 35,35',35",35"'; 45,45'; 55,55', 55"; 65,65'; 75,75',75") zur radialen Verbindung des radial inneren Ankoppelbereichs (14; 24; 34; 44; 54; 64; 74) mit radial äußeren Abschnitten (16; 26; 36,36'; 46,46'; 56,56'; 66,66'; 76,76') des ersten Befestigungselements (11; 21; 31; 41; 51; 61; 71) aufweist, wobei die radial äußeren Abschnitte des plattenförmigen ersten Befestigungselements einen äußeren Ringbereich bilden, der mindestens eine Unterbrechung (18; 28; 38; 48; 58; 68; 78) aufweist,
**dadurch gekennzeichnet,**
**dass** der Ankoppelbereich (14; 24; 34; 44; 54; 64; 74), die Stegelemente (15; 25,25',25"; 35,35',35",35"'; 45,45'; 55,55',55"; 65,65'; 75,75',75") und die radial äußeren Abschnitte (16; 26; 36,36'; 46,46'; 56,56'; 66,66'; 76,76') geometrisch derart gestaltet sind und ihr Material so gewählt ist, dass durch Auseinanderziehen bzw. Zusammenstauchen von Ankoppelbereich (14; 24; 34; 44; 54; 64; 74), Stegelementen (15; 25,25',25"; 35,35',35",35"'; 45,45'; 55,55',55"; 65,65'; 75,75',75") und radial äußeren Abschnitten (16; 26; 36,36'; 46,46'; 56,56'; 66,66'; 76,76') in der Plattenebene des plattenförmigen ersten Befestigungselements (11; 21; 31; 41; 51; 61; 71) eine bleibende plastische Verformung erfolgt, durch die der Außendurchmesser des ersten Befestigungselements (11; 21; 31; 41; 51; 61; 71) in diesem Bereich dauerhaft vergrößert bzw. verkleinert wird, und dass einander in der Ringebene gegenüber liegende radial äußere Abschnitte des plattenförmigen ersten Befestigungselements (11; 21; 31; 41; 51; 61; 71) jeweils einen die Ringebene des äußeren Ringbereichs durchstoßenden, in der Ringebene in, sich geschlossenen Durchbruch (19; 29; 39; 49; 59; 69; 79) aufweisen.

2. Gehörknöchelchenprothese nach Anspruch 1, **dadurch gekennzeichnet, dass** beide Befestigungselemente (21,22) plattenförmig und plastisch dauerhaft verformbar gestaltet sind, wobei das erste Befestigungselement (21) zur Anlage der Gehörknöchelchenprothese (20) auf der Steigbügelfußplatte ausgebildet ist und das zweite Befestigungselement (22) als ebene Kopfplatte zur mechanischen Verbindung mit dem Trommelfell dient.

3. Gehörknöchelchenprothese nach einem der vorhergehenden Ansprüche, **dadurch gekennzeichnet, dass** das plattenförmige erste Befestigungselement (11; 21; 31; 41; 51; 61; 71) eine Dicke, insbesondere eine Blechdicke t zwischen 0,01mm und 0,5mm sowie einen minimalen Durchmesser D zwischen 1,5mm und 8mm und die Stegelemente (15; 25,25',25"; 35,35',35",35"'; 45,45'; 55,55',55"; 65,65'; 75,75',75") eine maximale Breite b zwischen 0,01mm und 0,2mm aufweisen.

4. Gehörknöchelchenprothese nach einem der Ansprüche 1 bis 3, **dadurch gekennzeichnet, dass** der radial äußere Ringbereich des ersten Befestigungselements (11; 21; 31; 51; 61; 71) eine ovale oder kreisrunde Form aufweist.

5. Gehörknöchelchenprothese nach einem der Ansprüche 1 bis 3, **dadurch gekennzeichnet, dass** der radial äußere Ringbereich in der Plattenebene des plattenförmigen ersten Befestigungselements (41) eine radial nach außen verlaufende Ausbuchtung aufweist.

6. Gehörknöchelchenprothese nach einem der Ansprüche 1 bis 3, **dadurch gekennzeichnet, dass** der radial äußere Ringbereich des ersten Befestigungselements (61) eine einseitige Einbuchtung zur Aufnahme des Hammergriffs aufweist.

7. Gehörknöchelchenprothese nach einem der vorhergehenden Ansprüche, **dadurch gekennzeichnet, dass** die Stegelemente (15; 25'; 35"; 45'; 65') in der Plattenebene des plattenförmigen ersten Befestigungselements (11; 21; 31; 41; 61) nicht geradlinig, sondern auf - insbesondere mehrfach - gekrümmten Kurven verlaufen.

8. Gehörknöchelchenprothese nach einem der vorhergehenden Ansprüche, **dadurch gekennzeichnet, dass** jedes Stegelement (15; 25,25',25") mit mindestens zwei anderen Stegelementen verbunden ist.

9. Gehörknöchelchenprothese nach einem der vorhergehenden Ansprüche, **dadurch gekennzeichnet, dass** mindestens ein Verlängerungsteil (36'; 46'; 56'; 66'; 76') vorgesehen ist, das wie ein Puzzle-Teil am Außenrand des plattenförmigen ersten Befestigungselements (31; 41; 51; 61; 71) in der Plattenebene von der Seite her angeknöpft werden kann.

10. Gehörknöchelchenprothese nach Anspruch 9, **dadurch gekennzeichnet, dass** das seitlich anknöpfbare Verlängerungsteil (36'; 46'; 56'; 66'; 76') als Appendix für den Hammer oder den Hammergriff der Gehörknöchelchenprothese geformt ist und im zusammengeknöpften Zustand spitz vom Rand des plattenförmigen ersten Befestigungselements (31; 41; 51; 61; 71) radial nach außen wegragt.

11. Gehörknöchelchenprothese nach Anspruch 9 oder 10, **dadurch gekennzeichnet, dass** das seitlich anknöpfbare Verlängerungsteil (56'; 66'; 76') federnd im ersten Befestigungselement (51; 61; 71) verankert ist.

12. Gehörknöchelchenprothese nach Anspruch 11, **dadurch gekennzeichnet, dass** das seitlich anknöpfbare Verlängerungsteil (66'; 76') mittels eines Klammerelements im ersten Befestigungselement (61; 71) verankert ist.

13. Gehörknöchelchenprothese nach einem der Ansprüche 9 bis 12, **dadurch gekennzeichnet, dass** das seitlich anknöpfbare Verlängerungsteil (76') einrastend im ersten Befestigungselement (71) verankert ist, insbesondere mittels Widerhaken (77).

14. Gehörknöchelchenprothese nach einem der vorhergehenden Ansprüche, **dadurch gekennzeichnet, dass** das Verbindungselement (13; 23; 33) zwischen den beiden Befestigungselementen (11,12; 21,22; 31,32; 41,42; 51,42;.61,42; 71,32) als länglicher Schaft ausgeführt ist.

15. Gehörknöchelchenprothese nach Anspruch 14, **dadurch gekennzeichnet, dass** der längliche Schaft (13) mindestens ein Gelenk, vorzugsweise ein Kugelgelenk (17), insbesondere eine Kugelgelenk-Kette aufweist.

## Claims

1. An ossicular prosthetic (10; 20; 30; 40; 50; 60; 70) which replaces or bridges a link or parts of a link in the ossicular chain, wherein the ossicular prosthetic (10; 20; 30; 40; 50; 60; 70) comprises at its one end an essentially plate-shaped coupling element (11; 21; 31; 41; 51; 61; 71) for mounting on the eardrum or on the stirrup bone footplate, and at its other end a second coupling element (12; 22; 32; 42) for the mechanical connection to a link or parts of a link of the ossicular chain or to the inner ear, together with a connecting element (13; 23; 33) that with sound conduction interconnects the two coupling elements (11,12; 21,22; 31, 32; 41, 42; 51, 42; 61, 42; 71. 32), and wherein the plate-shaped first coupling element (11; 21; 31; 41; 51; 61; 71) has a radially inner coupling region (14; 24; 34; 44; 54; 64; 74) that is, in particular, arranged centrally about the centre of the area of the plate-shaped first coupling element (11; 21; 31; 41; 51; 61; 71) for the mechanical coupling of the first coupling element(11; 21; 31; 41; 51; 61; 71) to the connecting element (13; 23; 33), together with a plurality of web elements (15; 25, 25', 25"; 35, 35', 35", 35"', 45, 45'; 55, 55', 55"; 65, 65'; 75, 75', 75") for the radial connection of the radially inner coupling region (14; 24; 34; 44; 54; 64; 74) to radially outer sections (16; 26; 36, 36', 46, 46'; 56, 56'; 66,66'; 76, 76') of the first coupling element (11; 21; 31; 41; 51; 61; 71), and wherein the radially outer sections of the plate-shaped first coupling element form an outer annular region which has at least one interruption (18; 28; 38; 48; 58 68 78),
**characterised in that**
the coupling region (14; 24; 34; 44; 54; 64; 74), the web elements (15; 25, 25', 25"; 35, 35', 35", 35"'; 45, 45'; 55, 55', 55"; 65, 65'; 75, 75', 75") and the radially outer sections (16; 26; 36, 36'; 46, 46'; 56, 56'; 66, 66'; 76, 76') are geometrically shaped, and their material is selected in such a manner that a permanent plastic deformation takes place as a result of the expansion or contraction of the coupling region (14; 24; 34; 44; 54; 64; 74), the web elements (15; 25, 25', 25"; 35, 35', 35", 35"'; 45, 45'; 55, 55'; 65, 65'; 75, 75', 75") and the radially outer sections (16; 26; 36, 36'; 46, 46'; 56, 56'; 66, 66'; 76, 76') in the plate plane of the plate-shaped first coupling elements (11; 21; 31; 41; 51; 61; 71), as a result of which deformation the outside diameter of the first coupling element (11; 21; 31; 41; 51; 61; 71) is permanently increased and reduced, respectively, in this region, and **in that** radially outer sections of the plate-shaped first coupling element (11; 21; 31; 41; 51; 61; 71) opposing each other in the annular plane each have an opening (19; 29; 39; 49; 59; 69; 79) that penetrates the annular plane of the outer annular region and is closed in the annular plane.

2. The ossicular prosthetic according to Claim 1, **characterised in that** both coupling elements (21, 22) are of plate-shaped design with permanent plastic deformation, wherein the first coupling element (21) is designed for mounting the ossicular prosthetic (20) on the stirrup bone footplate, and **in that** the second coupling element (22) serves as a flat head plate for the mechanical connection to the eardrum.

3. The ossicular prosthetic according to any one of the preceding claims, **characterised in that** the plate-shaped first coupling element (11; 21; 31; 41; 51; 61; 71) has a thickness, in particular a plate thickness t of between 0.01 mm and 0.5 mm, and a minimum diameter D of between 1.5 mm and 8 mm, and **in that** the web elements (15; 25, 25', 25"; 35, 35', 35", 35"'; 45, 45'; 55, 55'; 65, 65'; 75, 75', 75") have a maximum width b of between 0.01 mm and 0.2 mm.

4. The ossicular prosthetic according to any one of Claims 1 to 3, **characterised in that** the radially outer annular region of the first coupling element (11; 21; 31; 51; 61; 71) has an oval or circular shape.

5. The ossicular prosthetic according to any one of Claims 1 to 3, **characterised in that** the radially outer annular region has an outward bulge running radially outwards in the plate plane of the plate-shaped first coupling element (41).

6. The ossicular prosthetic according to any one of Claims 1 to 3, **characterised in that** the radially outer annular region of the first coupling element (61) has a unilateral inward bulge for receiving the manubrium mallei.

7. The ossicular prosthetic according to any one of the preceding claims, **characterised in that** the web elements (15; 25', 35", 45'; 65') do not run rectilinearly, but along curves, in particular multiply bent curves, in the plate plane of the plate-shaped first coupling element (11; 21; 31; 41; 61).

8. The ossicular prosthetic according to any one of the preceding claims, **characterised in that** each web element (15; 25, 25', 25") is connected to at least two other web elements.

9. The ossicular prosthetic according to any one of the preceding claims, **characterised in that** at least one extension part (36'; 46'; 56'; 66'; 76') is provided which can be buttoned onto the outer edge of the plate-shaped first coupling element (31; 41; 51; 61; 71) from the side in the plate plane in the manner of a puzzle part.

10. The ossicular prosthetic according to Claim 9, **characterised in that** the extension part (36'; 46'; 56'; 66'; 76') that can be laterally buttoned on is shaped as an appendix for the malleus or the manubrium mallei of the ossicular prosthetic and, when buttoned up, projects sharply away from the edge of the plate-shaped first coupling element (31; 41; 51; 61; 71) in a radially outward direction.

11. The ossicular prosthetic according to Claim 9 or 10, **characterised in that** the extension part (56'; 66'; 76') that can be buttoned on laterally is resiliently anchored in the first coupling element (51; 61; 71).

12. The ossicular prosthetic according to Claim 11, **characterised in that** the extension part (66'; 76') that can be buttoned on laterally is anchored by means of a clamping element in the first coupling element.

13. The ossicular prosthetic according to any one of Claims 9 to 12, **characterised in that** the extension part (76') that can be buttoned on laterally is anchored so that it engages in the first coupling element (71), particularly by means of barbs (77).

14. The ossicular prosthetic according to any one of the preceding claims, **characterised in that** the connecting element (31; 23; 33) is constructed as an oblong shaft between the two coupling elements (11, 12; 21, 22; 31, 32; 41, 42; 51, 42; 61, 42; 71, 32).

15. The ossicular prosthetic according to Claim 14, **characterised in that** the oblong shaft (13) has at least one joint, preferably a ball joint (17), and in particular a ball joint chain.

## Revendications

1. Prothèse des osselets de l'oreille (10 ; 20 ; 30 ; 40 ; 50 ; 60 ; 70), qui remplace ou joint au moins un élément ou des parties d'un élément de la chaîne d'osselets de l'oreille, dans laquelle la prothèse des osselets de l'oreille (10 ; 20 ; 30 ; 40 ; 50 ; 60 ; 70) comprend, à une de ses extrémités, un premier élément de fixation (11 ; 21 ; 31 ; 41 ; 51 ; 61 ; 71) sensiblement en forme de plaque pour s'appuyer sur la membrane tympanique ou sur la plaque de base de l'étrier et, à son autre extrémité, un second élément de fixation (12 ; 22 ; 32 ; 42) pour se raccorder mécaniquement à un élément ou à des parties d'un élément de la chaîne d'osselets de l'oreille ou à l'oreille interne, ainsi qu'un élément de raccordement (13 ; 23 ; 33) reliant les deux éléments de fixation (11,12 ; 21,22 ; 31,32 ; 41,42 ; 51,42 ; 61,42 ; 71,32) l'un à l'autre de manière à conduire le son, et dans laquelle le premier élément de fixation (11 ; 21 ; 31 ; 41 ; 51 ; 61 ; 71) en forme de plaque présente une zone de couplage radialement interne (14 ; 24 ; 34 ; 44 ; 54 ; 64 ; 74) aménagée centralement en particulier autour du centre de gravité de surface du premier élément de fixation (11 ; 21 ; 31 ; 41 ; 51 ; 61 ; 71) en forme de plaque pour le couplage mécanique du premier élément de fixation (11 ; 21 ; 31 ; 41 ; 51 ; 61 ; 71) sur l'élément de raccord (13 ; 23 ; 33), ainsi que plusieurs traverses (15 ; 25,25',25" ; 35,35',35",35"' ; 45,45' ; 55,55', 55" ; 65,65' ; 75,75',75") pour le raccordement radial de la zone de couplage radialement interne (14 ; 24 ; 34 ; 44 ; 54 ; 64 ; 74) avec des sections radialement externes (16 ; 26 ; 36,36'; 46,46' ; 56,56' ; 66,66' ; 76,76') du premier élément de fixation (11 ; 21 ; 31 ; 41 ; 51 ; 61 ; 71), dans laquelle les sections radialement externes du premier élément de fixation en forme de plaque forment une zone annulaire externe qui présente au moins une interruption (18 ; 28 ; 38 ; 48 ; 58 ; 68 ; 78), **caractérisée en ce que** l'on façonne géométriquement la zone de couplage (14 ; 24 ; 34 ; 44 ; 54 ; 64 ; 74), les traverses (15 ; 25,25',25" ; 35,35',35",35"' ; 45,45' ; 55,55',55" ; 65,65' ; 75,75,75") et les sections radialement externes (16; 26 ; 36,36'; 46,46' ; 56,56' ; 66,66'; 76,76') et on choisit leur matériau de manière qu'il se produise, par écartement ou application conjointe de la zone de couplage (14 ; 24 ; 34 ; 44 ; 54 ; 64 ; 74), des traverses (15 ; 25,25',25" ; 35,35',35",35"' ; 45,45' ; 55,55',55" ; 65,65' ; 75,75',75") et des sections radialement externes (16 ; 26 ; 36,36' ; 46,46' ; 56,56' ; 66,66' ; 76,76') une déformation plastique permanente dans le plan de la plaque du premier élément de fixation (11 ; 21 ; 31 ; 41 ; 51 ; 61 ; 71) en forme de plaque, au moyen de laquelle le diamètre externe du premier élément de fixation (11 ; 21 ; 31 ; 41 ; 51 ; 61 ; 71) est agrandi ou diminué de manière permanente dans cette zone, et **en ce que** des sections radialement externes, situées l'une par rapport à l'autre dans le plan annulaire, du premier élément de fixation (11 ; 21 ; 31 ; 41 ; 51 ; 61 ; 71) en forme de plaque présentent respectivement un percement (19 ; 29 ; 39 ; 49 ; 59 ; 69 ; 79) passant à travers le plan annulaire de la zone annulaire externe et fermé en soi dans le plan annulaire.

2. Prothèse des osselets de l'oreille selon la revendication 1, **caractérisée en ce que** les deux éléments de fixation (21,22) sont conformés en plaque et de manière à présenter une déformation plastique permanente, dans laquelle le premier élément de fixation (21) est conformé pour appuyer la prothèse des osselets de l'oreille (20) sur la plaque de base de l'étrier et le second élément de fixation (22) sert de plaque de tête plane pour le raccordement mécanique avec la membrane tympanique.

3. Prothèse des osselets de l'oreille selon l'une quelconque des revendications précédentes, **caractérisée en ce que** le premier élément de fixation (11 ; 21 ; 31 ; 41 ; 51 ; 61 ; 71) en forme de plaque a une épaisseur, en particulier une épaisseur de tôle t comprise entre 0,01 mm et 0,5 mm, ainsi qu'un diamètre minimal D compris entre 1,5 mm et 8 mm et **en ce que** les traverses (15 ; 25,25',25" ; 35,35',35",35"' ; 45,45' ; 55,55',55" ; 65,65' ; 75,75',75") présentent une largeur maximale b comprise entre 0,01 mm et 0,2 mm.

4. Prothèse des osselets de l'oreille selon l'une quelconque des revendications 1 à 3, **caractérisée en ce que** la zone annulaire radialement externe du premier élément de fixation (11 ; 21 ; 31 ; 51 ; 61 ; 71) présente une forme ovale ou circulaire.

5. Prothèse des osselets de l'oreille selon l'une quelconque des revendications 1 à 3, **caractérisée en ce que** la zone annulaire radialement externe présente, dans le plan de la plaque du premier élément de fixation (41) en forme de plaque un renflement s'étendant radialement vers l'extérieur.

6. Prothèse des osselets de l'oreille selon l'une quelconque des revendications 1 à 3, **caractérisée en ce que** la zone annulaire radialement externe du premier élément de fixation (61) présente un renflement unilatéral pour recevoir le manche du marteau.

7. Prothèse des osselets de l'oreille selon l'une quelconque des revendications précédentes, **caractérisée en ce que** les traverses (15 ; 25' ; 35" ; 45' ; 65') ne s'étend pas de manière rectiligne dans le plan de la plaque du premier élément de fixation (11 ; 21 ; 31 ; 41 ; 61) en forme de plaque, mais sur des courbes incurvées - en particulier plusieurs fois.

8. Prothèse des osselets de l'oreille selon l'une quelconque des revendications précédentes, **caractérisée en ce que** chaque traverse (15 ; 25,25',25") est raccordée à au moins deux autres traverses.

9. Prothèse des osselets de l'oreille selon l'une quelconque des revendications précédentes, **caractérisée en ce qu'**il est prévu au moins une partie de prolongement (36' ; 46' ; 56' ; 66' ; 76') qui peut être engagée d'un côté à la manière d'une pièce de puzzle sur le bord externe du premier élément de fixation (31 ; 41 ; 51 ; 61 ; 71) en forme de plaque dans le plan de la plaque.

10. Prothèse des osselets de l'oreille selon la revendication 9, **caractérisée en ce que** la partie de prolongement (36' ; 46' ; 56' ; 66' ; 76'), qui peut être engagée latéralement, est formée en appendice pour le marteau ou le manche de marteau de la prothèse des osselets de l'oreille et, à l'état engagé, s'élève radialement vers l'extérieur en pointe depuis le bord du premier élément de fixation (31 ; 41 ; 51 ; 61 ; 71) en forme de plaque.

11. Prothèse des osselets de l'oreille selon la revendication 9 ou 10, **caractérisée en ce que** la partie de prolongement (56' ; 66' ; 76') qui peut être engagée latéralement est ancrée de manière élastique dans le premier élément de fixation (51; 61 ; 71).

12. Prothèse des osselets de l'oreille selon la revendication 11, **caractérisée en ce que** la partie de prolongement (66' ; 76') qui peut être engagée latéralement est ancrée au moyen d'un élément d'agrafage dans le premier élément de fixation (61 ; 71).

13. Prothèse des osselets de l'oreille selon l'une quelconque des revendications 9 à 12, **caractérisée en ce que** la partie de prolongement (76') qui peut être engagée latéralement est ancrée de manière encliquetable dans le premier élément de fixation (71), en particulier au moyen de barbes (77).

14. Prothèse des osselets de l'oreille selon l'une quelconque des revendications précédentes, **caractérisée en ce que** l'élément de raccordement (13 ; 23 ; 33) se présente entre les deux éléments de fixation (11,12 ; 21,22 ; 31,32 ; 41,42 ; 51,42 ; 61,42 ; 71,32) sous la forme d'une tige allongée.

15. Prothèse des osselets de l'oreille selon la revendication 14, **caractérisée en ce que** la tige allongée (13) présente au moins une articulation, de préférence une articulation à rotule (17), en particulier une chaîne d'articulations à rotule.
